(19) **Europäisches Patentamt**
**European Patent Office**
**Office européen des brevets**

(11) **EP 4 371 974 A1**

(12) **EUROPEAN PATENT APPLICATION**
published in accordance with Art. 153(4) EPC

(43) Date of publication:
**22.05.2024 Bulletin 2024/21**

(21) Application number: **22858520.4**

(22) Date of filing: **18.08.2022**

(51) International Patent Classification (IPC):
$C07C\ 263/10^{(2006.01)}$    $C07C\ 265/14^{(2006.01)}$
$C08G\ 18/70^{(2006.01)}$    $C25B\ 1/02^{(2006.01)}$
$C25B\ 1/26^{(2006.01)}$    $C25B\ 9/00^{(2021.01)}$
$C25B\ 9/65^{(2021.01)}$    $C25B\ 15/023^{(2021.01)}$
$C25B\ 15/08^{(2006.01)}$    $C07C\ 29/151^{(2006.01)}$
$C07C\ 31/04^{(2006.01)}$

(52) Cooperative Patent Classification (CPC):
**C07C 263/10; C07C 265/14; C08G 18/70;
C25B 1/02; C25B 1/26; C25B 9/00; C25B 9/65;
C25B 15/023; C25B 15/08;** C07C 29/151;
C07C 31/04

(86) International application number:
**PCT/JP2022/031260**

(87) International publication number:
**WO 2023/022203 (23.02.2023 Gazette 2023/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.08.2021 JP 2021134198
18.03.2022 JP 2022043980**

(71) Applicant: **Mitsui Chemicals, Inc.
Tokyo 105-7122 (JP)**

(72) Inventors:
• **TAKAI, Toshihiro
Tokyo 104-0028 (JP)**

• **SASAKI, Masaaki
Omuta-shi, Fukuoka 836-8610 (JP)**
• **MAEBA, Koji
Tokyo 104-0028 (JP)**
• **KURIHARA, Yasuyuki
Omuta-shi, Fukuoka 836-8610 (JP)**
• **KOBAYASHI, Tatsuhiko
Omuta-shi, Fukuoka 836-8610 (JP)**
• **YAMADA, Kunihiro
Omuta-shi, Fukuoka 836-8610 (JP)**
• **MORIOKA, Tsubasa
Omuta-shi, Fukuoka 836-8610 (JP)**

(74) Representative: **Hoffmann Eitle
Patent- und Rechtsanwälte PartmbB
Arabellastraße 30
81925 München (DE)**

(54) **ISOCYANATE PRODUCTION SYSTEM, ISOCYANATE COMPOSITION, POLYMERIZABLE COMPOSITION, RESIN, AND MOLDED ARTICLE**

(57) Provided is an isocyanate production system 1 including a control device 10 that selects energy produced by a biomass boiler 134, biomass methanol or carbon recycle methanol used by a methanol cracking device 150, recycled oil or biomass oil used by a carbon monoxide generation device 160, and a compound derived from a biomass raw material used by a polyamine production device 140 so that an environmental load value of an isocyanate compound, which is derived from energy produced by a boiler group 130, methanol used by the methanol cracking device 150, oil used by the carbon monoxide generation device 160, and a polyamine compound raw material used by the polyamine production device 140, is a predetermined value or less.

EP 4 371 974 A1

# FIG.1

1

┌─────────────────────────────┐
│ ISOCYANATE PRODUCTION       │ ～100
│         FACILITY            │
└─────────────────────────────┘
                ▲
                │
┌─────────────────────────────┐
│       CONTROL DEVICE        │ ～10
└─────────────────────────────┘

**Description**

Technical Field

The present disclosure relates to an isocyanate production system, an isocyanate composition, a polymerizable composition, a resin, and a molded article.

Background Art

Tolylene diisocyanate is widely known as a general-purpose isocyanate used as a raw material for polyurethane, polyurea, and the like. Tolylene diisocyanate is industrially produced from the reaction of diaminotoluene with phosgene (phosgene method) (see, for example, Patent Literature 1).

Citation List

Patent Literature

Patent Literature 1: Japanese Patent Application Laid-Open (JP-A) No. 2006-248998

SUMMARY OF INVENTION

Technical Problem

Energy is required in the process of producing an isocyanate compound such as tolylene diisocyanate, and carbon dioxide is discharged in the case of using fossil fuel for the production of the energy. Carbon dioxide is known as a greenhouse gas having a large influence on global warming, and it is required to produce an isocyanate compound in which the load on the environment is suppressed by reducing the emission amount of carbon dioxide.
The disclosure has been made in view of the above points, and an object thereof is to provide an isocyanate production system, an isocyanate composition, a polymerizable composition, a resin, and a molded article which contribute to reduction in the emission amount of carbon dioxide in the process of producing an isocyanate compound.

Solution to Problem

In order to solve the above problems, according to an aspect of the disclosure, there is provided an isocyanate production system including: at least one device selected from the group consisting of: a first production device that includes a biomass boiler producing energy used in the isocyanate production system, a second production device that produces carbon monoxide and hydrogen from methanol containing at least biomass methanol or carbon recycle methanol, a third production device that produces carbon monoxide and hydrogen from oil containing at least recycled oil or biomass oil, and a fourth production device that produces a polyamine compound from a polyamine compound raw material containing a biomass raw material; a fifth production device that produces phosgene from carbon monoxide and chlorine; a sixth production device that produces an isocyanate compound from a polyamine compound and the phosgene produced by the fifth production device; and a control device that controls the isocyanate production system, in which at least one of the carbon monoxide used in the fifth production device or the polyamine compound used in the sixth production device is produced by the at least one selected device, and the control device includes a selection unit, the selection unit selecting energy produced by the biomass boiler, the biomass methanol or carbon recycle methanol used by the second production device, the recycled oil or biomass oil used by the third production device, and a compound derived from the biomass raw material used by the fourth production device, so that an environmental load value of the isocyanate compound, which is derived from energy produced by the first production device, the methanol used by the second production device, the oil used by the third production device, and the polyamine compound raw material used by the fourth production device, is a predetermined value or less, in the at least one selected device.
The selection unit may select a use rate of each of the energy produced by the biomass boiler, the biomass methanol or carbon recycle methanol used by the second production device, the recycled oil or biomass oil used by the third production device, and the compound derived from the biomass raw material used by the fourth production device so as to achieve a target value of the environmental load value of the isocyanate compound.
The control device may further include a notification unit notifying the first production device to the sixth production device of an operating condition for satisfying the target value.
The environmental load value may be a carbon dioxide reduction credit certified according to a reduction amount of carbon dioxide.

The environmental load value may be an evaluation value obtained by evaluating an emission amount of carbon dioxide by production of the isocyanate compound by life cycle assessment.

The environmental load value may be a biomass plastic degree or biomass degree of the isocyanate compound.

The biomass raw material may contain at least one selected from the group consisting of biomass toluene, biomass benzene, biomass xylene, biomass naphthalene, and biomass dicyclopentadiene.

The polyamine compound produced from the polyamine compound raw material containing the biomass raw material may contain at least one selected from the group consisting of tolylenediamine, diaminodiphenylmethane, bis(aminocyclohexyl)methane, xylylenediamine, bis(aminomethyl)cyclohexane, naphthalenediamine, 2,5-bis(aminomethyl)bicyclo-[2.2.1]-heptane, and 2,6-bis(aminomethyl)bicyclo-[2.2.1]-heptane.

The isocyanate compound may contain at least one selected from the group consisting of tolylene diisocyanate, diphenylmethane diisocyanate, dicyclohexylmethane diisocyanate, xylylene diisocyanate, bis(isocyanatomethyl)cyclohexane, naphthalene diisocyanate, 2,5-bis(isocyanatomethyl)bicyclo-[2.2.1]-heptane, and 2,6-bis(isocyanatomethyl)bicyclo-[2.2.1]-heptane.

In order to solve the above problems, according to another aspect of the disclosure, there is provided an isocyanate composition containing an isocyanate compound derived from a biomass raw material.

In order to solve the above problems, according to still another aspect of the disclosure, there is provided an isocyanate composition containing an isocyanate compound having a biomass degree of 0.1% or more.

The biomass raw material may contain at least one selected from the group consisting of biomass toluene, biomass benzene, biomass xylene, biomass naphthalene, and biomass dicyclopentadiene.

The biomass raw material may be derived from a biomass naphtha raw material.

The isocyanate compound derived from a biomass raw material may contain at least one selected from the group consisting of tolylene diisocyanate derived from a biomass raw material, diphenylmethane diisocyanate derived from a biomass raw material, dicyclohexylmethane diisocyanate derived from a biomass raw material, xylylene diisocyanate derived from a biomass raw material, bis(isocyanatomethyl)cyclohexane derived from a biomass raw material, naphthalene diisocyanate derived from a biomass raw material, 2,5-bis(isocyanatomethyl)bicyclo-[2.2.1]-heptane derived from a biomass raw material, and 2,6-bis(isocyanatomethyl)bicyclo-[2.2.1]-heptane derived from a biomass raw material.

In order to solve the above problems, according to still another aspect of the disclosure, there is provided an isocyanate composition containing an isocyanate compound having a biomass degree of 0.1% or more.

A content of $^{14}C$ in the isocyanate compound may be $1 \times 10^{-13}\%$ or more.

The isocyanate compound may contain $^{14}C$ derived from at least one compound selected from the group consisting of $CO_2$ generated from an RPF boiler, $CO_2$ generated from a biomass boiler, biomass methanol, carbon recycle methanol, biomass oil, recycled oil, and biomass naphtha.

In order to solve the above problems, according to still another aspect of the disclosure, there is provided a polymerizable composition containing at least one of the isocyanate composition or a modified composition obtained by modifying the isocyanate composition.

The polymerizable composition may further contain an active hydrogen compound.

In order to solve the above problems, according to still another aspect of the disclosure, there is provided a resin which is a cured product of the polymerizable composition.

In order to solve the above problems, according to still another aspect of the disclosure, there is provided a molded article containing the resin.

In order to solve the above problems, according to still another aspect of the disclosure, there is provided an isocyanate production system including: a plurality of eleventh production devices that produce at least one of energy or carbon dioxide and each have a different environmental load value; a twelfth production device that produces hydrogen and chlorine by electrolyzing sodium chloride using at least one of at least some of the energy produced by the eleventh production devices or energy produced using renewable energy; a thirteenth production device that produces an alcohol using the carbon dioxide produced by the eleventh production devices and the hydrogen produced by the twelfth production device; a fourteenth production device that produces carbon monoxide and hydrogen from the alcohol produced by the thirteenth production device; a seventeenth production device that produces carbon monoxide and hydrogen from oil; a fifteenth production device that produces phosgene from the carbon monoxide produced by each of the fourteenth production device and the seventeenth production device and the chlorine produced by the twelfth production device; a sixteenth production device that produces an isocyanate compound from the phosgene produced by the fifteenth production device; and a control device that controls the isocyanate production system, in which the control device includes a selection unit, the selection unit selecting energy used by the eleventh production devices and the twelfth production device so that an environmental load value of the isocyanate compound, which is derived from energy or carbon dioxide produced by the plurality of eleventh production devices and energy used by the twelfth production device, is a predetermined value or less.

The plurality of eleventh production devices may include a first boiler using fossil fuel and at least one second boiler using fuel at least partially containing a plant-derived raw material.

The selection unit may select the eleventh production device by determining a use rate of each of the first boiler and the second boiler so that the environmental load value is the predetermined value or less.

The selection unit may determine a use rate of the energy produced using the renewable energy in the twelfth production device so that the environmental load value is the predetermined value or less.

The energy produced using the renewable energy and used in the twelfth production device may be energy produced by a solar power generation device.

The selection unit may select the eleventh production device using information on weather.

The selection unit may select a use rate of the energy produced using the renewable energy in the twelfth production device by using information on weather.

The seventeenth production device may produce carbon monoxide and hydrogen by using recycled oil for at least some of the oil. The selection unit may determine a use rate of recycled oil in the seventeenth production device so that the environmental load value of the isocyanate compound is the predetermined value or less.

The selection unit may select a use rate of the energy produced using the renewable energy so as to achieve a target value of the environmental load value of the isocyanate compound.

The selection unit may select the eleventh production device so as to achieve a target value of the environmental load value of the isocyanate compound.

The control device may further includes a target value calculation unit that calculates the target value based on a parameter related to the target value.

The parameter may be information on weather.

The parameter may be a target production amount of the phosgene to be produced.

The parameter may be a target production amount of the isocyanate compound to be produced.

The control device may further include a notification unit notifying the eleventh production devices to the sixteenth production device of an operating condition for satisfying the target value.

The environmental load value may be a carbon dioxide reduction credit certified according to a reduction amount of carbon dioxide.

The environmental load value may be an evaluation value obtained by evaluating an emission amount of carbon dioxide by production of the isocyanate compound by life cycle assessment.

The environmental load value may be a biomass plastic degree of the isocyanate compound.

The twelfth production device may produce hydrogen and chlorine by electrolyzing hydrogen chloride generated in a case in which the sixteenth production device produces an isocyanate compound.

The thirteenth production device may produce an alcohol using the hydrogen produced by the fourteenth production device or the seventeenth production device

The isocyanate compound may be tolylene diisocyanate or diphenylmethane diisocyanate.

Advantageous Effects of Invention

According to the disclosure, it is possible to provide an isocyanate production system, an isocyanate composition, a polymerizable composition, a resin, and a molded article which contribute to reduction in the emission amount of carbon dioxide in the process of producing an isocyanate compound.

BRIEF DESCRIPTION OF DRAWINGS

Fig. 1 is a diagram illustrating a schematic configuration of an isocyanate production system according to a first embodiment of the disclosed technology.
Fig. 2 is a diagram illustrating a configuration example of an isocyanate production facility.
Fig. 3 is a block diagram illustrating a hardware configuration of a control device.
Fig. 4 is a block diagram illustrating an example of a functional configuration of the control device.
Fig. 5 is a diagram illustrating a schematic configuration of an isocyanate production system according to a second embodiment of the disclosed technology.
Fig. 6 is a diagram illustrating a configuration example of an isocyanate production facility.
Fig. 7 is a block diagram illustrating a hardware configuration of a control device.
Fig. 8 is a block diagram illustrating an example of a functional configuration of the control device.

DESCRIPTION OF EMBODIMENTS

Hereinafter, an example of an embodiment of the disclosure will be described with reference to the drawings. In the drawings, the same or equivalent components and portions are denoted by the same reference numerals. Ratios of dimensions in the drawings may be exaggerated for convenience of explanation and thus may be different from actual ones.

(First Embodiment)

[Isocyanate Production System]

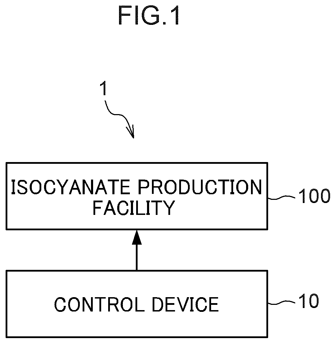

Fig. 1 is a diagram illustrating a schematic configuration of an isocyanate production system according to this embodiment.

An isocyanate production system 1 illustrated in Fig. 1 includes a control device 10 and an isocyanate production facility 100. The control device 10 is a device that controls the isocyanate production facility 100. The isocyanate production facility 100 includes a plurality of facilities and devices for producing isocyanate.

The isocyanate compound produced by the isocyanate production system according to this embodiment preferably contains at least one selected from the group consisting of tolylene diisocyanate, diphenylmethane diisocyanate, pentamethylene diisocyanate, hexamethylene diisocyanate, xylylene diisocyanate, naphthalene diisocyanate, isophorone diisocyanate, bis(isocyanatomethyl)cyclohexane, dicyclohexylmethane diisocyanate, 2,5-bis(isocyanatomethyl)bicyclo-[2.2.1]-heptane, and 2,6-bis(isocyanatomethyl)bicyclo-[2.2.1]-heptane, more preferably contains at least one selected from the group consisting of tolylene diisocyanate, diphenylmethane diisocyanate, xylylene diisocyanate, naphthalene diisocyanate, bis(isocyanatomethyl)cyclohexane, dicyclohexylmethane diisocyanate, 2,5-bis(isocyanatomethyl)bicyclo-[2.2.1]-heptane, and 2,6-bis(isocyanatomethyl)bicyclo-[2.2.1]-heptane, still more preferably contains at least one selected from the group consisting of tolylene diisocyanate and diphenylmethane diisocyanate, and still more preferably contains tolylene diisocyanate.

Hereinafter, the isocyanate production system according to this embodiment will be described focusing on the case of producing tolylene diisocyanate as a representative isocyanate compound.

Energy is required in the process of producing tolylene diisocyanate, but carbon dioxide is generated in the process of generating the energy. Carbon dioxide is known as a greenhouse gas having a large influence on global warming, and it is required to produce tolylene diisocyanate in which the load on the environment is suppressed by reducing the emission amount of carbon dioxide. Therefore, the control device 10 according to this embodiment makes it possible to produce tolylene diisocyanate in which the load on the environment is suppressed in the isocyanate production facility 100 that contributes to a reduction in the emission amount of carbon dioxide in the process of producing tolylene diisocyanate. The environmental load value may be a carbon dioxide reduction credit certified according to a reduction amount of carbon dioxide. The environmental load value may be an evaluation value obtained by evaluating an emission amount of carbon dioxide by production of the isocyanate compound by life cycle assessment. The environmental load value may be a green coefficient, or may be a biomass plastic degree or biomass degree of tolylene diisocyanate.

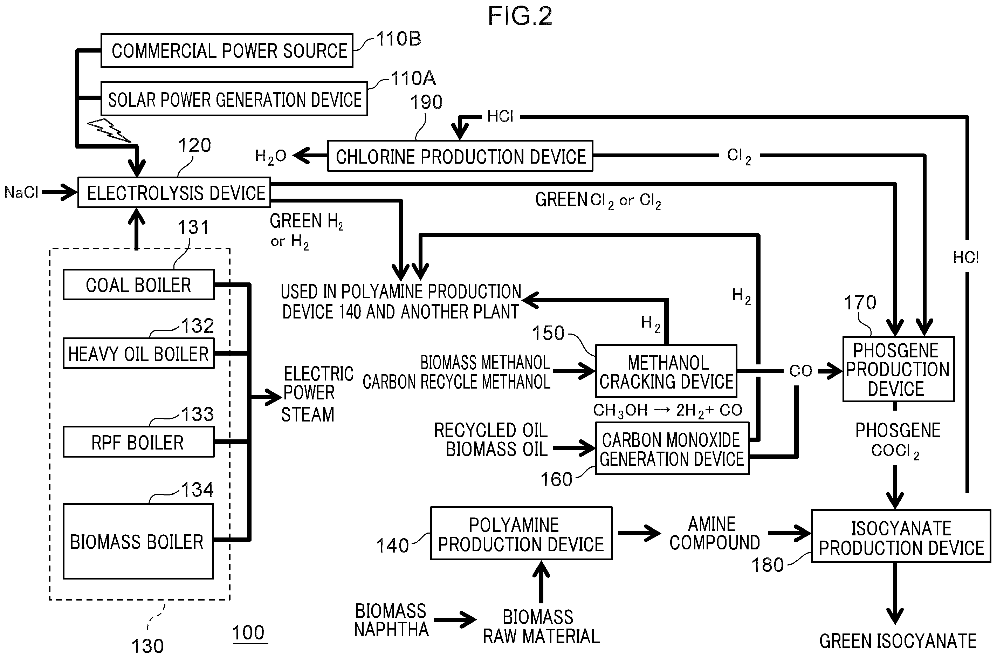

Fig. 2 is a diagram illustrating a configuration example of the isocyanate production facility 100.

The isocyanate production facility 100 includes an electrolysis device 120 that operates by receiving electric power from a solar power generation device 110A, a commercial power source 110B, or a boiler group 130, the boiler group 130, a polyamine production device 140, a methanol cracking device 150, a carbon monoxide generation device 160, a phosgene production device 170, and an isocyanate production device 180.

The electrolysis device 120 is a device that electrolyzes sodium chloride (NaCl) to produce chlorine (Ch) and hydrogen (Hz). As described above, the electrolysis device 120 operates by receiving electric power from the solar power generation device 110A, the commercial power source 110B, or the boiler group 130.

The boiler group 130 includes a plurality of boilers having different environmental load values. The fact that the environmental load values are different means that the degree of influence on the natural environment varies depending on difference in fuel to be used. In Fig. 2, as the boiler group 130, a coal boiler 131, a heavy oil boiler 132, an RPF boiler 133, and a biomass boiler 134 are illustrated. Each of the boilers is an example of the first production device of the disclosure. The coal boiler 131 is a boiler that burns coal to generate energy. The heavy oil boiler 132 is a boiler that burns at least one of heavy oil, light oil, or fuel oil to generate energy. The RPF boiler 133 is a boiler that burns refuse paper and plastic fuel (RPF, mainly, solid fuel containing, as raw materials, waste paper and plastics which are difficult to recycle among industrial wastes) to generate energy. The biomass boiler 134 is a boiler that burns biomass fuel to generate energy. Each boiler generates electric power or steam as energy.

In this embodiment, the coal boiler 131 has the highest environmental load value, the heavy oil boiler 132 has the second highest environmental load value, the RPF boiler 133 has the third highest environmental load value, and the biomass boiler 134 has the lowest environmental load value.

The polyamine production device 140 produces a polyamine compound from a polyamine compound raw material containing a biomass raw material. The polyamine production device 140 may produce a polyamine compound not containing a biomass raw material from a polyamine compound raw material not containing a biomass raw material. That is, the polyamine production device 140 may produce a polyamine compound raw material from petroleum that is fossil fuel, and then produce a polyamine compound. The polyamine production device 140 is an example of the fourth production device of the disclosure.

The biomass raw material is not particularly limited, but for example, a biomass raw material containing at least one selected from the group consisting of biomass toluene, biomass benzene, biomass xylene, biomass naphthalene, and biomass dicyclopentadiene is used.

As the biomass raw material, a biomass naphtha raw material produced from biomass naphtha (hereinafter also referred to as "biomass raw material derived from a biomass naphtha raw material") is preferred.

The biomass raw material produced from biomass naphtha preferably contains at least one selected from the group consisting of biomass toluene produced from biomass naphtha, biomass benzene produced from biomass naphtha, biomass xylene produced from biomass naphtha, biomass naphthalene produced from biomass naphtha, and biomass dicyclopentadiene produced from biomass naphtha.

The polyamine compound produced from the polyamine compound raw material containing the biomass raw material contains, for example, at least one selected from the group consisting of tolylenediamine, diaminodiphenylmethane, bis(aminocyclohexyl)methane, xylylenediamine, bis(aminomethyl)cyclohexane, naphthalenediamine, 2,5-bis(aminomethyl)bicyclo-[2.2.1]-heptane, and 2,6-bis(aminomethyl)bicyclo-[2.2.1]-heptane.

The methanol cracking device 150 is a device that cracks and/or reforms methanol to produce carbon monoxide and hydrogen from methanol. The methanol cracking device 150 is an example of the second production device of the disclosure.

The methanol cracked by the methanol cracking device 150 includes, for example, carbon recycle methanol, which is methanol generated from carbon monoxide and hydrogen, and/or biomass methanol, which is methanol produced from a biomass raw material. The methanol cracked by the methanol cracking device 150 may include methanol derived from petroleum or natural gas in addition to the carbon recycle methanol or the biomass methanol. The methanol cracked by the methanol cracking device 150 may be at least one of the various kinds of methanol exemplified above, or may be a mixture of a plurality of the various kinds of methanol.

The carbon monoxide generated by the methanol cracking device 150 is used for production of phosgene in the phosgene production device 170.

The carbon monoxide generation device 160 is a device that allows oil, oxygen and steam to react at a high temperature to generate carbon monoxide and hydrogen. The carbon monoxide generation device 160 is an example of the third production device of the disclosure.

The oil used in the carbon monoxide generation device 160 includes, for example, recycled oil and/or biomass oil which is oil produced from a biomass raw material.

The oil used in the carbon monoxide generation device 160 may be at least one of crude oil, heavy oil, light oil, or fuel oil, in addition to the recycled oil and the biomass oil.

The oil used in the carbon monoxide generation device 160 may be a mixture of a plurality of the various kinds of oil exemplified above.

Examples of the recycled oil include waste edible oil, waste engine oil, waste lubricating oil, waste glycerin, recycled heavy oil, recycled biodiesel oil, pyrolytic oil of waste plastic, and waste rubber-derived pyrolytic oil. The hydrogen generated by each of the electrolysis device 120, the methanol cracking device 150, and the carbon monoxide generation device 160 is used in another plant or the polyamine production device 140.

The phosgene production device 170 is a device that produces phosgene ($COCl_2$) using carbon monoxide generated by the methanol cracking/reforming device 150 and/or the carbon monoxide generation device 160 and chlorine generated by the electrolysis device 120. The phosgene production device 170 is an example of the fifth production device of the disclosure.

The isocyanate production device 180 produces tolylene diisocyanate using the phosgene produced by the phosgene production device 170. Tolylene diisocyanate is produced by reacting tolylenediamine with phosgene. The isocyanate production device 180 is an example of the sixth production device of the disclosure. At least one of the carbon monoxide or chlorine used in the phosgene production device 170 or the polyamine compound used in the isocyanate production device 180 is produced by at least one device selected from the group consisting of each boiler of the boiler group 130, the electrolysis device 120, the polyamine production device 140, the methanol cracking device 150, the carbon monoxide generation device 160, and a chlorine production device 190.

The isocyanate production facility 100 may include the chlorine production device 190. The chlorine production device 190 is a device that oxidizes hydrogen chloride (HCl) generated in the process of producing tolylene diisocyanate by the isocyanate production device 180 to produce chlorine. The chlorine produced by the chlorine production device 190 may be used for production of phosgene in the phosgene production device 170.

In addition to the solar power generation device 110A or instead of the solar power generation device 110A, a power generation device using renewable energy other than sunlight may be used.

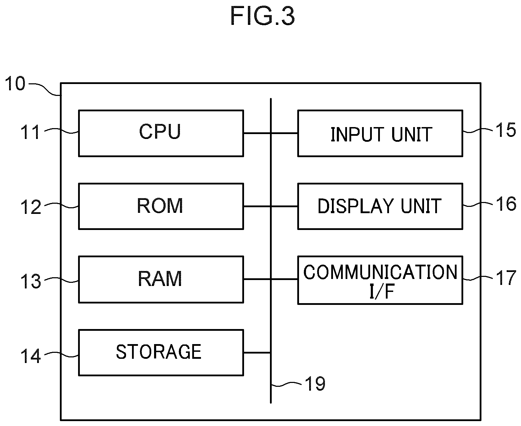

Fig. 3 is a block diagram illustrating a hardware configuration of the control device 10.

As illustrated in Fig. 3, the control device 10 includes a central processing unit (CPU) 11, a read only memory (ROM) 12, a random access memory (RAM) 13, a storage 14, an input unit 15, a display unit 16, and a communication interface (I/F) 17. The respective components are communicably connected to each other via a bus 19.

The CPU 11 is a central processing unit, and executes various programs or controls each unit. That is, the CPU 11 reads a program from the ROM 12 or the storage 14 and executes the program using the RAM 13 as a workspace. The CPU 11 performs control of each of the components and various types of arithmetic processing according to a program recorded in the ROM 12 or the storage 14. In this embodiment, the ROM 12 or the storage 14 stores a control program for controlling the isocyanate production facility 100.

The ROM 12 stores various programs and various data. The RAM 13 temporarily stores a program or data as a workspace. The storage 14 includes a storage device such as a hard disk drive (HDD), a solid state drive (SSD), or a flash memory, and stores various programs including an operating system and various data.

The input unit 15 includes pointing device such as a mouse and a keyboard, and is used to perform various inputs.

The display unit 16 is, for example, a liquid crystal display, and displays various types of information. The display unit 16 may function as the input unit 15 by employing a touch panel system.

The communication interface 17 is an interface for communicating with another device such as the isocyanate production facility 100, and employs, for example, a standard such as Ethernet (registered trademark), FDDI, or Wi-Fi (registered trademark).

When executing the control program, the control device 10 implements various functions by using the hardware resources. A functional configuration implemented by the control device 10 will be described.

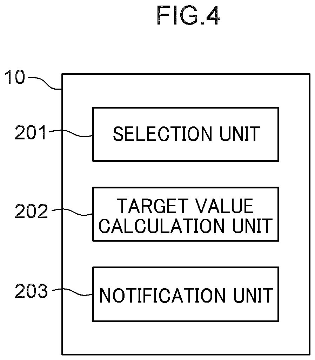

Fig. 4 is a block diagram illustrating an example of a functional configuration of the control device 10.

As illustrated in Fig. 4, the control device 10 includes, as functional configurations, a selection unit 201, a target value calculation unit 202, and a notification unit 203. Each functional configuration is realized by the CPU 11 reading out and executing the control program stored in the ROM 12 or the storage 14.

The selection unit 201 selects each boiler of the boiler group 130 so that an environmental load value of the isocyanate compound, which is derived from carbon dioxide produced by each boiler of the boiler group 130 and energy used by the electrolysis device 120, is a predetermined value or less. The selection unit 201 selects a compound derived from a biomass raw material used in a case in which the polyamine production device 140 produces a polyamine compound. The selection unit 201 determines a rate of the biomass methanol in the methanol cracked by the methanol cracking device 150. The selection unit 201 determines a rate of the recycled oil or biomass oil in the oil used by the carbon monoxide generation device 160 so that the environmental load value is the predetermined value or less. The selection unit 201 may determine a use rate of the solar power generation device 110A or the commercial power source 110B so that the environmental load value is the predetermined value or less.

The selection unit 201 may select each boiler of the boiler group 130, the compound derived from the biomass raw material used in a case in which the polyamine production device 140 produces a polyamine compound, a rate of the biomass methanol in the methanol cracked by the methanol cracking device 150, and a rate of the recycled oil or biomass oil in the oil used by the carbon monoxide generation device 160 so as to achieve a predetermined target value of the environmental load value of the isocyanate compound.

The target value calculation unit 202 calculates the target value based on a parameter related to the target value. For example, the target value calculation unit 202 may use information on a target production amount of the isocyanate compound to be produced as a parameter. In a case in which the target production amount of the isocyanate compound is large, the target value calculation unit 202 may perform calculation in the direction of increasing the target value of the environmental load value. For example, the target value calculation unit 202 may use information on a target production amount of the phosgene to be produced as a parameter. In a case in which the target production amount of the phosgene is large, the target value calculation unit 202 may perform calculation in the direction of increasing the target value of the environmental load value.

The notification unit 203 notifies each device of the isocyanate production facility 100 of an operating condition for satisfying the target value. Each device of the isocyanate production facility 100 can achieve the target value of the environmental load value of the isocyanate compound to be produced by operating based on the operating condition notified from the notification unit 203.

Since the control device 10 has the functional configuration illustrated in Fig. 4, it is possible to control the isocyanate production facility 100 that effectively uses carbon dioxide generated in the energy generation process and suppresses the load on the environment.

The isocyanate production system according to above-described embodiment is used for production of the isocyanate compound without any particular limitation. Preferred examples of the isocyanate compound are as described above.

The isocyanate production system according to above-described embodiment is also suitably used for an isocyanate composition according to the following embodiment.

[Isocyanate Composition]

The isocyanate composition according to this embodiment is an isocyanate composition of the following first aspect or second aspect.

<Isocyanate Composition of First Aspect>

An isocyanate composition of a first aspect contains an isocyanate compound derived from a biomass raw material.

The isocyanate composition of the first aspect may have the characteristics and/or preferred characteristics of an isocyanate composition of a second aspect described below.

In the isocyanate composition of the first aspect, the biomass raw material preferably contains at least one selected from the group consisting of biomass toluene, biomass benzene, biomass xylene, biomass naphthalene, and biomass dicyclopentadiene.

In the isocyanate composition of the first aspect, the biomass raw material is preferably a biomass raw material derived from a biomass naphtha raw material. In the isocyanate composition of the first aspect, the biomass raw material may be carbon monoxide. In this case, the carbon monoxide may be carbon monoxide obtained by decomposing the carbon recycle methanol or the biomass methanol. Phosgene ($COCl_2$) derived from a biomass raw material may be produced using the carbon monoxide and the chlorine, and an isocyanate compound derived from a biomass raw material may be produced by reacting the phosgene with a polyamine compound not derived from a biomass raw material.

In the isocyanate composition of the first aspect, the isocyanate compound derived from a biomass raw material preferably contains at least one selected from the group consisting of tolylene diisocyanate derived from a biomass raw material, diphenylmethane diisocyanate derived from a biomass raw material, dicyclohexylmethane diisocyanate derived from a biomass raw material, xylylene diisocyanate derived from a biomass raw material, bis(isocyanatomethyl)cyclohexane derived from a biomass raw material, naphthalene diisocyanate derived from a biomass raw material, 2,5-bis(isocyanatomethyl)bicyclo-[2.2.1]-heptane derived from a biomass raw material, and 2,6-bis(isocyanatomethyl)bicyclo-[2.2.1]-heptane derived from a biomass raw material.

<Isocyanate Composition of Second Aspect>

An isocyanate composition of a second aspect contains an isocyanate compound having a biomass degree of 0.1% or more.

The biomass degree of the isocyanate compound contained in the isocyanate composition of the second aspect is preferably 0.5% or more and more preferably 1% or more.

The isocyanate composition of the second aspect may have the characteristics and/or preferred characteristics of the isocyanate composition of the first aspect described above.

In the disclosure, the biomass degree is a value determined by a carbon-based calculation method and calculated on the basis of the following formula.

Biomass degree (%) = {(Number of carbon atoms derived from plant)/(Number of carbon atoms derived from plant + Number of carbon atoms derived from petroleum)} $\times$ 100

For example, in a case in which tolylene diisocyanate is synthesized using phosgene from tolylenediamine, the biomass degree of the tolylene diisocyanate can be calculated on the basis of the following formula.

Biomass degree of tolylene diisocyanate (%) = {[(Number of carbon atoms of tolylenediamine $\times$ Biomass degreee of tolylenediamine)+(Number of carbon atoms of phosgene $\times$ Biomass degreee of phosgene)]/(Number of carbon atoms of tolylenediamine + Number of carbon atoms of phosgene)} $\times$ 100

In the disclosure, the biomass degree (%) can be calculated by measuring a reference value difference of a radioactive carbon ($^{14}C$) content from $CO_2$ generated by burning a measurement sample by accelerator mass spectrometry (AMS) in accordance with a test method of American Standard Test Method (ASTM) D6866 (Standard Test Method for Determining the Biobased Content of Natural Range Materials Using Radiocarbon and Isotope Ratio Mass Spectrometry Analysis).

In the isocyanate compound in the isocyanate composition of the second aspect, the content of $^{14}C$ (radioactive carbon) is preferably $1 \times 10^{-13}\%$ or more, more preferably $5 \times 10^{-13}\%$ or more, and still more preferably $1 \times 10^{-12}\%$ or more. The content of $^{14}C$ (radioactive carbon) is usually $1 \times 10^{-10}\%$ or less.

The content (%) of $^{14}C$ means a ratio (number%) of $^{14}C$ in a case in which the number of all carbon atoms (that is, $^{12}C$ and $^{14}C$) contained in the isocyanate compound is taken as 100%, that is, a value represented by the following formula.

$$^{14}\text{C content (\%)} = \text{Number of }^{14}\text{C}/(\text{Number of }^{14}\text{C} + \text{Number of }^{12}\text{C}) \times 100$$

For the content (%) of $^{14}C$, Netsu Sokutei 39 (4), 143-150 (2012) (hereinafter, referred to as Literature A) can be referred to.

As described in Literature A, in a case in which all C in the isocyanate compound is derived from a plant, the $^{14}C$ content (%) is $1 \times 10^{-10}$ (%).

In a case in which all C in the isocyanate compound is C derived from fossil fuel such as petroleum or coal, the number of $^{14}C$ is 0, and thus the $^{14}C$ content (%) is 0%.

As described above, the $^{14}C$ content (%) in the isocyanate compound can be determined by determining the number of $^{14}C$ in the isocyanate compound by the test method of ASTM B6866.

The isocyanate compound having a content (%) of $^{14}C$ in the above preferred range can be produced by the isocyanate production system according to this embodiment described above.

The content (%) of $^{14}C$ in a commercially available isocyanate compound (that is, an isocyanate compound not derived from a biomass raw material) is substantially 0% (less than $1 \times 10^{-13}\%$ at most) (see, for example, urethane comprehensive site "PU Portal" (https://pu-portal.com/blog/1294/)).

The isocyanate compound in the isocyanate composition of the second aspect preferably contains $^{14}C$ derived from at least two compounds selected from the group consisting of $CO_2$ generated from an RPF boiler, $CO_2$ generated from a biomass boiler, biomass methanol, carbon recycle methanol, biomass oil, recycled oil, and biomass naphtha.

"RPF" in the RPF boiler means "Refuse derived paper and plastics densified fuel" defined in JIS Z 7311:2010.

[Polymerizable Composition]

A polymerizable composition according to this embodiment contains at least one of the isocyanate composition according to the above-described embodiment or an isocyanate-modified composition obtained by modifying the isocyanate composition.

The isocyanate-modified composition is a composition produced by modifying the isocyanate composition according to the above-described embodiment.

The isocyanate-modified composition contains at least one of the following functional groups (a) to (i):

(a) an isocyanurate group,
(b) an allophanate group,
(c) a biuret group,
(d) a urethane group,
(e) a urea group,
(f) a carbodiimide group,
(g) an iminooxadiazinedione group,
(h) a uretdione group, and
(i) a uretonimine group.

The isocyanate-modified composition may contain at least one of the above functional groups (a) to (i), and can contain two or more kinds thereof.

The isocyanate-modified composition can be used singly, or in combination of two or more kinds thereof.

The isocyanate-modified composition may be used in combination with the isocyanate composition according to the above-described embodiment (that is, the composition before modification).

The polymerizable composition according to this embodiment preferably further contains an active hydrogen compound.

Examples of the active hydrogen compound include

polyols (that is, compounds having two or more hydroxyl groups),
polythiols (that is, compound having two or more mercapto groups (that is, thiol groups)), and
polyamines (that is, compounds having two or more amino groups).

The polymerizable composition according to this embodiment may contain only one active hydrogen compound or two or more active hydrogen compounds.

The active hydrogen compound may be an active hydrogen compound derived from a biomass raw material, or may be an active hydrogen compound not derived from a biomass raw material.

The active hydrogen compound may contain only one of an active hydrogen compound derived from a biomass raw material or an active hydrogen compound not derived from a biomass raw material, or may contain both of them.

The active hydrogen compound may contain a known monool, a known monothiol, a known monoamine, or the like.

[Resin and Molded Article]

A resin according to this embodiment is a cured product of the polymerizable composition according to the above-described embodiment.

The resin according to this embodiment is obtained by curing the polymerizable composition according to this embodiment.

A molded article according to this embodiment contains the resin according to the above-described embodiment.

The resin according to this embodiment preferably is a reaction product of

at least one of the isocyanate composition according to the above-described embodiment or the isocyanate-modified composition according to the above-described embodiment, and

the above-described active hydrogen compound.

The resin (or the molded article) according to this embodiment can be used for all use applications in which a poly(thio)urethane resin is used.

The poly(thio)urethane resin means a polyurethane resin or a polythiourethane resin.

Examples of use applications of the resin (or the molded article) according to this embodiment include inks, transfer foils, pressure-sensitive adhesives, binders, gels, elastomers, foams, one-component curable sealants, RIM molded articles, microcellular polyurethane, various microcapsules, optical materials, aqueous resins, thermosetting resins, active energy curable resins, artificial and synthetic leathers, slush powder, robot members, mobility members, health care materials, substrate resins for carbon-fiber reinforced plastic (CFRP), transparent rubbers, transparent hard resins, waterproof materials, films, sheets, tubes, blades, speakers, sensors, organic EL members, solar power generation members, android members, wearable members, sporting goods, leisure goods, medical products, nursing care goods, housing materials, acoustic materials, lighting members, chandeliers, street lights, gaskets, vibration proofing and damping/base isolation members, sound insulation materials, daily use articles, miscellaneous goods, cushions, beddings, stress absorbing materials, stress relieving materials, automobile interior and exterior members, OA device members, surface protection members for miscellaneous goods, self-repairing materials, and healthcare products.

For use applications of the resin (or the molded article) according to this embodiment, for example, the description in paragraphs 0277 to 0373 of WO 2018/190290 A may be appropriately referred to.

Hereinafter, each of the optical material, the foam, and the elastomer, which are examples of use applications of the resin (or the molded article) according to this embodiment, will be described in detail.

<Optical Material>

Examples of the optical material include:

optical lenses such as transparent lenses, sunglass lenses, polarizing lenses, eyeglass lenses, camera lenses, pick-up lenses, and contact lenses;

vehicle lighting panels, headlight lenses, lamp covers of headlight and rear light, and optical members such as optical elements, optical discs, organic ELs, LEDs; and

illumination for signboard, optical fibers, glass alternatives, intermediate films for laminated glass, windproof materials for aircrafts and the like, large water tank walls, transparent roof materials, glazing materials, transparent members for daily use articles, protection glasses, hood, protective shields, automotive safety components, lighting components, and optical products such as smartphones and tablets.

Among the optical materials, optical lenses are preferred.

The optical material (preferably optical lenses) preferably contains a reaction product of the isocyanate composition according to this embodiment and/or the isocyanate-modified composition with a polythiol as an active hydrogen compound.

The polythiol to be used for production of the optical material (preferably optical lenses) may be used singly or in combination of two or more kinds thereof.

The polythiol to be used for production of the optical material (preferably optical lenses) may contain only one of or both of a polythiol derived from a biomass raw material and a polythiol not derived from a biomass raw material.

Examples of the polythiol include aliphatic polythiols containing a sulfur atom other than a sulfur atom in a mercapto group.

Examples of such aliphatic polythiols include 1,2-bis [(2-mercaptoethyl)thio]-3-mercaptopropane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, and mixtures thereof.

Examples of a method of producing the optical material include a one-shot method.

As described above, the optical material is preferably produced by using, as raw materials, the polyisocyanate composition according to this embodiment and/or the isocyanate-modified composition and a polythiol compound as an active hydrogen compound.

In the process of producing the optical material, components other than the above raw materials may be blended.

Examples of the other components include urethane-forming catalysts, internal release agents (for example, a phosphoric acid ester-based release agent, an alkyl phosphate-based release agent, a fatty acid ester-based release agent, and the like), ultraviolet absorbers (for example, a benzotriazole compound, a formamidine-based compound, and the like), blueing agents, plasticizers, antifoaming agents, leveling agents, delusterants, fire retardants, thixotropic agents, tackifiers, thickening agents, lubricants, antistatic agents, surfactants, reaction retardants, dehydrators, antioxidants, hydrolysis inhibitors, and weathering stabilizers.

\<Foam\>

Examples of the foam include a soft foam and a hard foam.

The hardness (25% CLD) of the soft foam is, for example, less than 40.6 N/100 cm$^2$, and the hardness (25% CLD) of the hard foam is, for example, 40.6 N/100 cm$^2$ or more.

The foam preferably contains a reaction product of the isocyanate composition according to this embodiment and/or the isocyanate-modified composition with a high-molecular-weight polyol as an active hydrogen compound and a blowing agent. Each of the high-molecular-weight polyol compound and the blowing agent may be used singly or in combination of two or more kinds thereof.

Examples of the high-molecular-weight polyol include polyether polyols.

The high-molecular-weight polyol to be used for production of the optical material (preferably optical lenses) may contain only one of or both of a high-molecular-weight polyol derived from a biomass raw material and a high-molecular-weight polyol not derived from a biomass raw material.

Examples of the blowing agent include chemical blowing agents (for example, water) and physical blowing agents (for example, methylenechlorides, chlorofluorocarbons, hydroxychlorofluorocarbons, carbon dioxide, an organic blowing agent, an inorganic blowing agent, and the like).

The foam can be produced by, for example, a known foaming method.

Specifically, components (for example, a high-molecular-weight polyol and a blowing agent) other than the isocyanate composition and the isocyanate-modified composition are blended in advance to prepare a resin premix. Then, the isocyanate composition and/or the isocyanate-modified composition is blended with the resin premix and foam-molded to produce foam.

For the foam-molding, for example, known methods such as a slab foaming method and a mold foaming method are used. The foaming can also be performed by a mechanical froth foam molding method.

As described above, the foam is preferably produced by using, as raw materials, the isocyanate composition according to this embodiment and/or the isocyanate-modified composition, a high-molecular-weight polyol as an active hydrogen compound, and a blowing agent.

In the process of producing the foam, components other than the above raw materials may be blended.

Examples of the other components include urethane-forming catalysts, cross-linking agents, foam stabilizers, heat-resistant stabilizers (antioxidants), light stabilizers, and multifunctional stabilizers.

\<Elastomer\>

Examples of the elastomer include thermoplastic urethane elastomer (TPU) and thermosetting urethane elastomer (TSU).

The elastomer may be used, for example, as a component in a coating agent or an adhesive.

The elastomer includes, for example, a soft segment and a hard segment.

The soft segment contains, for example, a reaction product of the isocyanate composition according to this embodiment and/or the isocyanate-modified composition with a high-molecular-weight polyol as an active hydrogen compound.

The hard segment contains, for example, a reaction product of the isocyanate composition according to this embodiment and/or the isocyanate-modified composition with a low-molecular-weight polyol and/or low-molecular-weight polyamine as an active hydrogen compound.

Examples of the high-molecular-weight polyol include polyester polyols (for example, polycaprolactone polyol, adipate-based polyester polyol (polyester polyol using adipic acid as polybasic acid)), polycarbonate polyols, and polytetramethylene ether glycol (for example, polytetramethylene ether glycol), and adipate-based polyester polyol is preferred.

Examples of the low-molecular-weight polyol include ethylene glycol and 1,4-butylene glycol, and 1,4-butylene glycol is preferred.

The elastomer can be produced by, for example, a known method such as a one-shot method or a prepolymer method.

In the one-shot method, for example, the isocyanate composition according to this embodiment and/or the isocyanate-modified composition, a high-molecular-weight polyol, and a low-molecular-weight polyol and/or low-molecular-weight polyamine are allowed to react all at once to produce an elastomer.

In the prepolymer method, for example, first, the isocyanate composition according to this embodiment and/or the isocyanate-modified composition is allowed to react with a high-molecular-weight polyol to synthesize an isocyanate group-terminated prepolymer having an isocyanate group at its molecular terminal. Then, the obtained isocyanate group-terminated prepolymer is allowed to react with a low-molecular-weight polyol and/or low-molecular-weight polyamine to produce an elastomer.

For the method of producing an elastomer, for example, a bulk polymerization method, a solution polymerization method, or the like can be applied.

As described above, the elastomer is preferably produced by using, as raw materials, the isocyanate composition according to this embodiment and/or the isocyanate-modified composition, a high-molecular-weight polyol as an active hydrogen compound, and a low-molecular-weight polyol and/or low-molecular-weight polyamine as an active hydrogen compound.

In the process of producing the elastomer, components other than the above raw materials may be blended.

Examples of the other components include urethane-forming catalysts, plasticizers, anti-blocking agents, heat-resistant stabilizers, light stabilizers, ultraviolet absorbers, NOx yellowing inhibitors, antioxidants, release agents, pigments, dyes, lubricants, nucleating agents, fillers, and hydrolysis inhibitors.

(Second Embodiment)

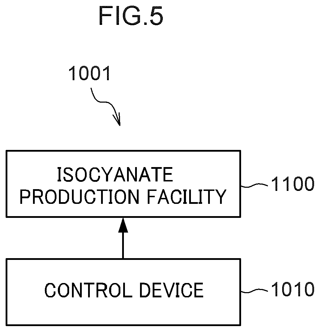

Fig. 5 is a diagram illustrating a schematic configuration of an isocyanate production system according to this embodiment.

An isocyanate production system 1001 illustrated in Fig. 5 includes a control device 1010 and an isocyanate production facility 1100. The control device 1010 is a device that controls the isocyanate production facility 1100. The isocyanate production facility 1100 includes a plurality of facilities and devices for producing isocyanate. The isocyanate compound to be produced is preferably tolylene diisocyanate, diphenylmethane diisocyanate, pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, and 2,6-bis(isocyanatomethyl)bicyclo-[2.2.1]-heptane, and more preferably tolylene diisocyanate and diphenylmethane diisocyanate. In this embodiment, the isocyanate production facility 1100 that produces tolylene diisocyanate as a representative isocyanate compound is illustrated.

Energy is required in the process of producing tolylene diisocyanate, but carbon dioxide is generated in the process of generating the energy. Carbon dioxide is known as a greenhouse gas having a large influence on global warming, and it is required to produce tolylene diisocyanate in which the load on the environment is suppressed by effectively using carbon dioxide. Therefore, the control device 1010 according to this embodiment makes it possible to produce tolylene diisocyanate in which the load on the environment is suppressed in the isocyanate production facility 1100 by effectively utilizing carbon dioxide generated from a plurality of boilers having different environmental load values in the process of producing tolylene diisocyanate. The environmental load value may be a carbon dioxide reduction credit certified according to a reduction amount of carbon dioxide. The environmental load value may be an evaluation value obtained by evaluating an emission amount of carbon dioxide by production of the isocyanate compound by life cycle assessment. The environmental load value may be a green coefficient, or may be a biomass plastic degree of tolylene diisocyanate.

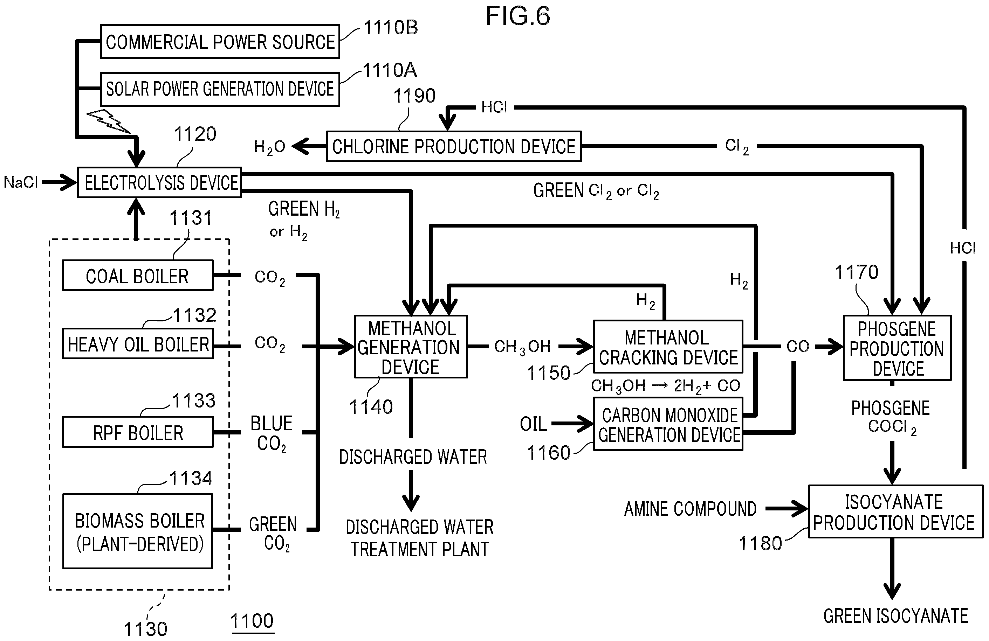

Fig. 6 is a diagram illustrating a configuration example of an isocyanate production facility 1100.

The isocyanate production facility 1100 includes an electrolysis device 1120 that operates by receiving electric power from a solar power generation device 1110A, a commercial power source 1110B, or a boiler group 1130, the boiler group 1130, a methanol generation device 1140, a methanol cracking device 1150, a carbon monoxide generation device 1160, a phosgene production device 1170, and an isocyanate production device 1180.

The electrolysis device 1120 is a device that electrolyzes sodium chloride (NaCl) to produce chlorine (Ch) and hydrogen ($H_2$). The electrolysis device 1120 is an example of the twelfth production device of the disclosure. As described above, the electrolysis device 120 operates by receiving electric power from the solar power generation device 1110A, the commercial power source 1110B, or the boiler group 1130.

The boiler group 1130 includes a plurality of boilers having different environmental load values. The fact that the environmental load values are different means that the degree of influence on the natural environment varies depending on difference in fuel to be used. In Fig. 6, as the boiler group 1130, a coal boiler 1131, a heavy oil boiler 1132, an RPF boiler 1133, and a biomass boiler 1134 are illustrated. Each of the boilers is an example of the eleventh production device of the disclosure. The coal boiler 1131 is a boiler that burns coal to generate energy. The heavy oil boiler 1132 is a boiler that burns at least one of heavy oil, light oil, or fuel oil to generate energy. The RPF boiler 1133 is a boiler that burns refuse paper and plastic fuel (RPF, mainly, solid fuel containing, as raw materials, waste paper and plastics which are difficult to recycle among industrial wastes) to generate energy. The biomass boiler 1134 is a boiler that burns biomass fuel to generate energy.

In this embodiment, the coal boiler 1131 has the highest environmental load value, the heavy oil boiler 1132 has the second highest environmental load value, the RPF boiler 1133 has the third highest environmental load value, and the biomass boiler 1134 has the lowest environmental load value.

The methanol generation device 1140 is a device that generates methanol ($CH_3OH$) from hydrogen and carbon dioxide. The methanol generation device 1140 is an example of the thirteenth production device of the disclosure. The methanol generation device 1140 generates methanol by using the hydrogen produced by the electrolysis device 1120 and the carbon dioxide discharged from each boiler of the boiler group 1130. The methanol generation device 140 produces methanol from hydrogen and carbon dioxide by a chemical immobilization process using a catalyst. For example, the methanol generation device 1140 produces methanol by using a technique disclosed in WO 2011/136345 A, WO 2012/067222 A, or the like. The methanol generation device 1140 may generate methanol by using the hydrogen generated by the methanol cracking device 1150 in addition to the hydrogen produced by the electrolysis device 1120.

The methanol cracking device 1150 is a device that cracks and/or reforms the methanol generated by the methanol generation device 1140 to produce carbon monoxide and hydrogen from methanol. The methanol cracking device 1150 is an example of the fourteenth production device of the disclosure. The carbon monoxide generated by the methanol cracking device 150 is used for production of phosgene in the phosgene production device 1170. The hydrogen generated by the methanol cracking device 1150 may be used for generation of methanol in the methanol generation device 1140.

The carbon monoxide generation device 1160 is a device that allows oil, oxygen and steam to react at a high temperature to generate carbon monoxide and hydrogen. The oil used here may be at least one of crude oil, heavy oil, light oil, fuel oil, or recycled oil, or may be a mixture of two or more thereof. Examples of the recycled oil include waste edible oil, waste engine oil, waste lubricating oil, waste glycerin, recycled heavy oil, recycled biodiesel oil, pyrolytic oil of waste plastic, and waste rubber-derived pyrolytic oil. The carbon monoxide generation device 1160 is an example of the seventeenth production device of the disclosure. The hydrogen generated by the carbon monoxide generation device 1160 may be used for generation of methanol in the methanol generation device 1140.

The phosgene production device 1170 is a device that produces phosgene ($COCl_2$) using carbon monoxide generated by the methanol cracking/reforming device 1150 and/or the carbon monoxide generation device 1160 and chlorine generated by the electrolysis device 1120. The phosgene production device 1170 is an example of the fifteenth production device of the disclosure.

The isocyanate production device 1180 produces tolylene diisocyanate using the phosgene produced by the phosgene production device 1170. The isocyanate production device 1180 is an example of the sixteenth production device of the disclosure. Tolylene diisocyanate is produced by reacting tolylenediamine with phosgene.

The isocyanate production facility 1100 may include a chlorine production device 1190. The chlorine production device 1190 is a device that oxidizes hydrogen chloride (HCl) generated in the process of producing tolylene diisocyanate by the isocyanate production device 1180 to produce chlorine. The chlorine produced by the chlorine production device 1190 may be used for production of phosgene in the phosgene production device 1170.

In addition to the solar power generation device 1110A or instead of the solar power generation device 1110A, a power generation device using renewable energy other than sunlight may be used.

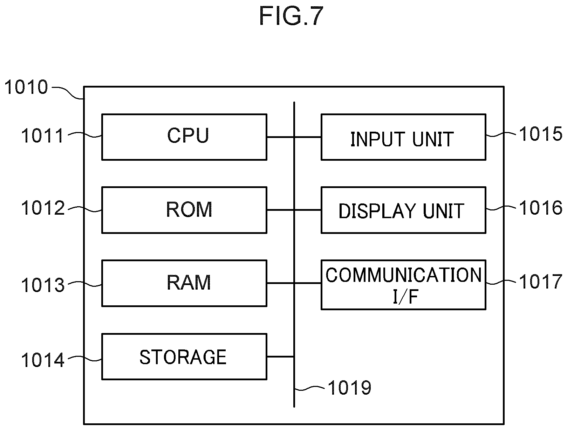

Fig. 7 is a block diagram illustrating a hardware configuration of the control device 1010.

As illustrated in Fig. 7, the control device 1010 includes a central processing unit (CPU) 1011, a read only memory (ROM) 1012, a random access memory (RAM) 1013, a storage 1014, an input unit 1015, a display unit 1016, and a communication interface (I/F) 1017. The respective components are communicably connected to each other via a bus 1019.

The CPU 1011 is a central processing unit, and executes various programs or controls each unit. That is, the CPU 1011 reads a program from the ROM 1012 or the storage 1014 and executes the program using the RAM 1013 as a workspace. The CPU 1011 performs control of each of the components and various types of arithmetic processing according to a program recorded in the ROM 1012 or the storage 1014. In this embodiment, the ROM 1012 or the storage 1014 stores a control program for controlling the isocyanate production facility 1100.

The ROM 1012 stores various programs and various data. The RAM 1013 temporarily stores a program or data as a workspace. The storage 1014 includes a storage device such as a hard disk drive (HDD), a solid state drive (SSD), or a flash memory, and stores various programs including an operating system and various data.

The input unit 1015 includes pointing device such as a mouse and a keyboard, and is used to perform various inputs.

The display unit 1016 is, for example, a liquid crystal display, and displays various types of information. The display unit 1016 may function as the input unit 1015 by employing a touch panel system.

The communication interface 1017 is an interface for communicating with another device such as the isocyanate production facility 1100, and employs, for example, a standard such as Ethernet (registered trademark), FDDI, or Wi-Fi (registered trademark).

When executing the control program, the control device 1010 implements various functions by using the hardware resources. A functional configuration implemented by the control device 1010 will be described.

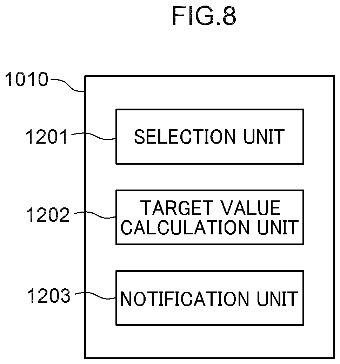

Fig. 8 is a block diagram illustrating an example of a functional configuration of the control device 1010.

As illustrated in Fig. 8, the control device 1010 includes, as functional configurations, a selection unit 1201, a target value calculation unit 1202, and a notification unit 1203. Each functional configuration is realized by the CPU 1011 reading out and executing the control program stored in the ROM 1012 or the storage 1014.

The selection unit 1201 determines a use rate of the solar power generation device 1110A or the commercial power source 1110B so that the environmental load value is the predetermined value or less. The selection unit 1201 selects each boiler of the boiler group 1130 so that an environmental load value of the isocyanate compound, which is derived from carbon dioxide produced by each boiler of the boiler group 1130 and energy used by the electrolysis device 1120, is a predetermined value or less.

The selection unit 1201 may select each boiler of the boiler group 1130 by determining a use rate of each boiler of the boiler group 1130 so that the environmental load value is the predetermined value or less. For example, an environmental load value of the coal boiler 1131 is designated as A, an environmental load value of the heavy oil boiler 1132 is designated as B, an environmental load value of the RPF boiler 1133 is designated as C, and an environmental load value of the biomass boiler 1134 is designated as D. The selection unit 1201 may select each boiler of the boiler group 1130 by determining a use rate of each boiler of the boiler group 1130 so that the sum of the respective environmental load values is the predetermined value or less. The selection unit 1201 may determine a rate of the recycled oil in the oil used by the carbon monoxide production device 1160 so that the environmental load value is the predetermined value or less.

The selection unit 1201 may determine a use rate of the energy produced using the renewable energy in the electrolysis device 1120 so that the environmental load value is the predetermined value or less. That is, the selection unit 1201 may determine a use rate of the solar power generation device 1110A or the commercial power source 1110B in the electrolysis device 1120 so that the environmental load value is the predetermined value or less.

The selection unit 1201 may select the solar power generation device 1110A or the commercial power source 110B by determining a use rate of the solar power generation device 1110A or the commercial power source 1110B by using information on weather. For example, in a case in which it is expected that the amount of power generation by the solar power generation device 1110A will be stabilized due to continuous good weather, the selection unit 1201 may increase the use rate of the solar power generation device 1110A in order to reduce the environmental load value. In the case of increasing the use rate of the solar power generation device 1110A in order to reduce the environmental load value, the selection unit 1201 may increase the use rate of the coal boiler 1131 in order to use a large amount of energy and carbon dioxide derived from the coal boiler 1131.

The selection unit 1201 may select each boiler of the boiler group 1130, a rate of the recycled oil used by the carbon monoxide production device 1160, or the solar power generation device 1110A or the commercial power source 1110B so as to achieve a predetermined target value of the environmental load value of the isocyanate compound.

The target value calculation unit 1202 calculates the target value based on a parameter related to the target value. For example, the target value calculation unit 1202 may use information on weather as a parameter. A weather forecast in a predetermined future period may be used as the information on weather. In a case in which a sunny day is equal to or more than a predetermined ratio in a predetermined period, the target value calculation unit 1202 may perform calculation in the direction of decreasing the target value of the environmental load value. For example, the target value calculation unit 1202 may use information on a target production amount of the isocyanate compound to be produced as a parameter. In a case in which the target production amount of the isocyanate compound is large, the target value calculation unit 1202 may perform calculation in the direction of increasing the target value of the environmental load value. For example, the target value calculation unit 1202 may use information on a target production amount of the phosgene to be produced as a parameter. In a case in which the target production amount of the phosgene is large, the target value calculation unit 1202 may perform calculation in the direction of increasing the target value of the environmental load value.

The notification unit 1203 notifies each device of the isocyanate production facility 1100 of an operating condition for satisfying the target value. Each device of the isocyanate production facility 1100 can achieve the target value of the environmental load value of the isocyanate compound to be produced by operating based on the operating condition notified from the notification unit 1203.

Since the control device 1010 has the functional configuration illustrated in Fig. 8, it is possible to control the isocyanate production facility 1100 that effectively uses carbon dioxide generated in the energy generation process and suppresses the load on the environment.

The control program executed by the CPU in each of the embodiments may be executed by various processors other than the CPU. Examples of the processor in this case include a programmable logic device (PLD) such as a field-programmable gate array (FPGA) in which a circuit configuration can be changed after manufacturing, and a dedicated electric circuit such as an application specific integrated circuit (ASIC), which is a processor having a circuit configuration exclusively designed to perform specific processing. The control program may be executed by one of these various processors, or may be executed by any combination of two or more processors of the same type or different types (for example, any combination of a plurality of FPGAs, any combination of a CPU and an FPGA, or the like). More specifically, the hardware structure of these various processors is an electric circuit in which circuit elements such as semiconductor elements are combined.

Each of the embodiments has described the aspect in which the control program is stored (installed) in advance the ROM or the storage, but the invention is not limited thereto. The program may be provided in a form of being recorded in a non-transitory recording medium such as a compact disk read only memory (CD-ROM), a digital versatile disk read only memory (DVD-ROM), or a universal serial bus (USB) memory. The program may be downloaded from an external device through a network.

The entire contents of the disclosures by Japanese Patent Application No. 2021-134198 filed on August 19, 2021 and Japanese Patent Application No. 2022-043980 filed on March 18, 2022 are incorporated herein by reference.

All the literature, patent application, and technical standards cited herein are also herein incorporated to the same extent as provided for specifically and severally with respect to an individual literature, patent application, and technical standard to the effect that the same should be so incorporated by reference.

**Claims**

1. An isocyanate production system comprising:

   at least one device selected from the group consisting of:

   a first production device that includes a biomass boiler producing energy used in the isocyanate production system,
   a second production device that produces carbon monoxide and hydrogen from methanol containing at least biomass methanol or carbon recycle methanol,
   a third production device that produces carbon monoxide and hydrogen from oil containing at least recycled oil or biomass oil, and
   a fourth production device that produces a polyamine compound from a polyamine compound raw material containing a biomass raw material;
   a fifth production device that produces phosgene from carbon monoxide and chlorine;
   a sixth production device that produces an isocyanate compound from a polyamine compound and the phosgene produced by the fifth production device; and
   a control device that controls the isocyanate production system,
   wherein at least one of the carbon monoxide used in the fifth production device or the polyamine compound used in the sixth production device is produced by the at least one selected device, and
   the control device includes a selection unit, the selection unit selecting energy produced by the biomass boiler, the biomass methanol or carbon recycle methanol used by the second production device, the recycled oil or biomass oil used by the third production device, and a compound derived from the biomass raw material used by the fourth production device, so that an environmental load value of the isocyanate compound, which is derived from energy produced by the first production device, the methanol used by the second production device, the oil used by the third production device, and the polyamine compound raw material used by the fourth production device, is a predetermined value or less, in the at least one selected device.

2. The isocyanate production system according to claim 1, wherein the selection unit selects a use rate of each of the energy produced by the biomass boiler, the biomass methanol or carbon recycle methanol used by the second production device, the recycled oil or biomass oil used by the third production device, and the compound derived from the biomass raw material used by the fourth production device so as to achieve a target value of the environmental load value of the isocyanate compound.

3. The isocyanate production system according to claim 2, wherein the control device further includes a notification unit notifying the first production device to the sixth production device of an operating condition for satisfying the target value.

4. The isocyanate production system according to any one of claims 1 to 3, wherein the environmental load value is a carbon dioxide reduction credit certified according to a reduction amount of carbon dioxide.

5. The isocyanate production system according to any one of claims 1 to 4, wherein the environmental load value is an evaluation value obtained by evaluating an emission amount of carbon dioxide by production of the isocyanate compound by life cycle assessment.

6. The isocyanate production system according to any one of claims 1 to 5, wherein the environmental load value is a biomass plastic degree or biomass degree of the isocyanate compound.

7. The isocyanate production system according to any one of claims 1 to 6, wherein the biomass raw material contains at least one selected from the group consisting of biomass toluene, biomass benzene, biomass xylene, biomass naphthalene, and biomass dicyclopentadiene.

8. The isocyanate production system according to any one of claims 1 to 7, wherein the polyamine compound produced from the polyamine compound raw material containing the biomass raw material contains at least one selected from the group consisting of tolylenediamine, diaminodiphenylmethane, bis(aminocyclohexyl)methane, xylylenediamine, bis(aminomethyl)cyclohexane, naphthalenediamine, 2,5-bis(aminomethyl)bicyclo-[2.2.1]-heptane, and 2,6-bis(aminomethyl)bicyclo-[2.2.1]-heptane.

9. The isocyanate production system according to any one of claims 1 to 8, wherein the isocyanate compound contains at least one selected from the group consisting of tolylene diisocyanate, diphenylmethane diisocyanate, dicyclohexylmethane diisocyanate, xylylene diisocyanate, bis(isocyanatomethyl)cyclohexane, naphthalene diisocyanate, 2,5-bis(isocyanatomethyl)bicyclo-[2.2.1]-heptane, and 2,6-bis(isocyanatomethyl)bicyclo-[2.2.1]-heptane.

10. An isocyanate composition comprising an isocyanate compound derived from a biomass raw material.

11. The isocyanate composition according to claim 10, wherein the biomass raw material contains at least one selected from the group consisting of biomass toluene, biomass benzene, biomass xylene, biomass naphthalene, and biomass dicyclopentadiene.

12. The isocyanate composition according to claim 10 or 11, wherein the biomass raw material is derived from a biomass naphtha raw material.

13. The isocyanate composition according to any one of claims 10 to 12, wherein the isocyanate compound derived from a biomass raw material contains at least one selected from the group consisting of tolylene diisocyanate derived from a biomass raw material, diphenylmethane diisocyanate derived from a biomass raw material, dicyclohexylmethane diisocyanate derived from a biomass raw material, xylylene diisocyanate derived from a biomass raw material, bis(isocyanatomethyl)cyclohexane derived from a biomass raw material, naphthalene diisocyanate derived from a biomass raw material, 2,5-bis(isocyanatomethyl)bicyclo-[2.2.1]-heptane derived from a biomass raw material, and 2,6-bis(isocyanatomethyl)bicyclo-[2.2.1]-heptane derived from a biomass raw material.

14. An isocyanate composition comprising an isocyanate compound having a biomass degree of 0.1% or more.

**15.** The isocyanate composition according to any one of claims 10 to 14, wherein a content of $^{14}C$ in the isocyanate compound is $1 \times 10^{-13}$% or more.

**16.** The isocyanate composition according to any one of claims 10 to 15, wherein the isocyanate compound contains $^{14}C$ derived from at least one compound selected from the group consisting of $CO_2$ generated from an RPF boiler, $CO_2$ generated from a biomass boiler, biomass methanol, carbon recycle methanol, biomass oil, recycled oil, and biomass naphtha.

**17.** A polymerizable composition comprising at least one of the isocyanate composition according to any one of claims 10 to 16 or an isocyanate-modified composition obtained by modifying the isocyanate composition.

**18.** The polymerizable composition according to claim 17, further comprising an active hydrogen compound.

**19.** A resin which is a cured product of the polymerizable composition according to claim 17 or 18.

**20.** A molded article comprising the resin according to claim 19.

**21.** An isocyanate production system comprising:

a plurality of eleventh production devices that produce at least one of energy or carbon dioxide and each have a different environmental load value;

a twelfth production device that produces hydrogen and chlorine by electrolyzing sodium chloride using at least one of at least some of the energy produced by the eleventh production devices or energy produced using renewable energy;

a thirteenth production device that produces an alcohol using the carbon dioxide produced by the eleventh production devices and the hydrogen produced by the twelfth production device;

a fourteenth production device that produces carbon monoxide and hydrogen from the alcohol produced by the thirteenth production device;

a seventeenth production device that produces carbon monoxide and hydrogen from oil;

a fifteenth production device that produces phosgene from the carbon monoxide produced by each of the fourteenth production device and the seventeenth production device and the chlorine produced by the twelfth production device;

a sixteenth production device that produces an isocyanate compound from the phosgene produced by the fifteenth production device; and

a control device that controls the isocyanate production system,

wherein the control device includes a selection unit, the selection unit selecting energy used by the eleventh production devices and the twelfth production device so that an environmental load value of the isocyanate compound, which is derived from energy or carbon dioxide produced by the plurality of eleventh production devices and energy used by the twelfth production device, is a predetermined value or less.

**22.** The isocyanate production system according to claim 21, wherein the plurality of eleventh production devices include a first boiler using fossil fuel and at least one second boiler using fuel at least partially containing a plant-derived raw material.

**23.** The isocyanate production system according to claim 22, wherein the selection unit selects the eleventh production device by determining a use rate of each of the first boiler and the second boiler so that the environmental load value is the predetermined value or less.

**24.** The isocyanate production system according to claim 21, wherein the selection unit determines a use rate of the energy produced using the renewable energy in the twelfth production device so that the environmental load value is the predetermined value or less.

**25.** The isocyanate production system according to claim 24, wherein the energy produced using the renewable energy and used in the twelfth production device is energy produced by a solar power generation device.

**26.** The isocyanate production system according to claim 25, wherein the selection unit selects the eleventh production device using information on weather.

27. The isocyanate production system according to claim 24, wherein the selection unit selects a use rate of the energy produced using the renewable energy in the twelfth production device by using information on weather.

28. The isocyanate production system according to claim 21, wherein the seventeenth production device produces carbon monoxide and hydrogen by using recycled oil for at least some of the oil.

29. The isocyanate production system according to claim 26, wherein the selection unit determines a use rate of recycled oil in the seventeenth production device so that the environmental load value of the isocyanate compound is the predetermined value or less.

30. The isocyanate production system according to any one of claims 21 to 27, wherein the selection unit selects a use rate of the energy produced using the renewable energy so as to achieve a target value of the environmental load value of the isocyanate compound.

31. The isocyanate production system according to any one of claims 21 to 27, wherein the selection unit selects the eleventh production device so as to achieve a target value of the environmental load value of the isocyanate compound.

32. The isocyanate production system according to claim 30 or 31, wherein the control device further includes a target value calculation unit that calculates the target value based on a parameter related to the target value.

33. The isocyanate production system according to claim 32, wherein the parameter is information on weather.

34. The isocyanate production system according to claim 32, wherein the parameter is a target production amount of the phosgene to be produced.

35. The isocyanate production system according to claim 32, wherein the parameter is a target production amount of the isocyanate compound to be produced.

36. The isocyanate production system according to any one of claims 30 to 35, wherein the control device further includes a notification unit notifying the eleventh production devices to the sixteenth production device of an operating condition for satisfying the target value.

37. The isocyanate production system according to any one of claims 21 to 36, wherein the environmental load value is a carbon dioxide reduction credit certified according to a reduction amount of carbon dioxide.

38. The isocyanate production system according to any one of claims 21 to 36, wherein the environmental load value is an evaluation value obtained by evaluating an emission amount of carbon dioxide by production of the isocyanate compound by life cycle assessment.

39. The isocyanate production system according to any one of claims 21 to 36, wherein the environmental load value is a biomass plastic degree of the isocyanate compound.

40. The isocyanate production system according to any one of claims 21 to 39, wherein the twelfth production device produces hydrogen and chlorine by electrolyzing hydrogen chloride generated in a case in which the sixteenth production device produces an isocyanate compound.

41. The isocyanate production system according to any one of claims 21 to 40, wherein the thirteenth production device produces an alcohol using the hydrogen produced by the fourteenth production device or the seventeenth production device.

42. The isocyanate production system according to any one of claims 21 to 41, wherein the isocyanate compound is tolylene diisocyanate or diphenylmethane diisocyanate.

FIG.1

1

```
┌─────────────────────────────┐
│ ISOCYANATE PRODUCTION       │ ～100
│         FACILITY            │
└─────────────────────────────┘
              ▲
              │
┌─────────────────────────────┐
│       CONTROL DEVICE        │ ～10
└─────────────────────────────┘
```

# FIG.2

FIG.3

10

| 11 | CPU | | INPUT UNIT | 15 |
| 12 | ROM | | DISPLAY UNIT | 16 |
| 13 | RAM | | COMMUNICATION I/F | 17 |
| 14 | STORAGE | | | |

19

# FIG.4

10 —

201 — SELECTION UNIT

202 — TARGET VALUE CALCULATION UNIT

203 — NOTIFICATION UNIT

FIG.5

# FIG.6

EP 4 371 974 A1

# FIG.7

1010
1011 CPU
1012 ROM
1013 RAM
1014 STORAGE
1015 INPUT UNIT
1016 DISPLAY UNIT
1017 COMMUNICATION I/F
1019

# FIG.8

1010

1201 — SELECTION UNIT

1202 — TARGET VALUE CALCULATION UNIT

1203 — NOTIFICATION UNIT

## INTERNATIONAL SEARCH REPORT

| International application No. |
|---|
| **PCT/JP2022/031260** |

**A. CLASSIFICATION OF SUBJECT MATTER**

*C07C 263/10*(2006.01)i; *C07C 265/14*(2006.01)i; *C08G 18/70*(2006.01)i; *C25B 1/02*(2006.01)i; *C25B 1/26*(2006.01)i; *C25B 9/00*(2021.01)i; *C25B 9/65*(2021.01)i; *C25B 15/023*(2021.01)i; *C25B 15/08*(2006.01)i; *C07C 29/151*(2006.01)n; *C07C 31/04*(2006.01)n

FI:  C07C263/10; C25B1/26 A; C25B1/02; C25B15/023; C25B15/08 302; C25B9/65; C25B9/00 F; C25B9/00 Z; C07C265/14; C08G18/70; C07C31/04; C07C29/151

According to International Patent Classification (IPC) or to both national classification and IPC

**B. FIELDS SEARCHED**

Minimum documentation searched (classification system followed by classification symbols)

C07C263/00; C07C265/00; C08G18/00; C25B1/00; C25B9/00; C25B9/00; C25B15/00; C07C29/00; C07C31/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2006-248998 A (MITSUI CHEMICALS POLYURETHANES INC) 21 September 2006 (2006-09-21) claims 1-9 | 1-42 |
| A | JP 2007-023034 A (BAYER MATERIALSCIENCE AG) 01 February 2007 (2007-02-01) claims 1-22 | 1-42 |
| X | WO 2015/060260 A1 (MITSUI CHEMICALS, INC) 30 April 2015 (2015-04-30) claims 1-23, paragraphs [0015], [0017] | 10-20 |
| A | | 1-9, 21-42 |
| X | JP 2019-099605 A (SAKATA INKS) 24 June 2019 (2019-06-24) claims 1-6, paragraph [0052] | 10-20 |
| X | JP 2019-142029 A (DAINIPPON PRINTING CO LTD) 29 August 2019 (2019-08-29) claims 1-18, paragraph [0083] | 10-20 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 November 2022** | **15 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/031260**

C.      DOCUMENTS CONSIDERED TO BE RELEVANT

| Category* | Citation of document, with indication, where appropriate, of the relevant passages | Relevant to claim No. |
|---|---|---|
| X | JP 2014-520086 A (BASF SE) 21 August 2014 (2014-08-21)<br>claims 1-18 | 10-20 |
| X | JP 2004-123666 A (TOYOTA MOTOR CORP) 22 April 2004 (2004-04-22)<br>claims 1-3, paragraphs [0013]-[0016] | 10-20 |

Form PCT/ISA/210 (second sheet) (January 2015)

**INTERNATIONAL SEARCH REPORT**
Information on patent family members

International application No.

**PCT/JP2022/031260**

| Patent document cited in search report | | | Publication date (day/month/year) | Patent family member(s) | | | Publication date (day/month/year) |
|---|---|---|---|---|---|---|---|
| JP | 2006-248998 | A | 21 September 2006 | US 2008/0154066 A1 claims 1-23 WO 2006/095761 A1 EP 1857438 A1 CN 101142173 A KR 10-2013-0120551 A | | | |
| JP | 2007-023034 | A | 01 February 2007 | US 2007/0012577 A1 claims 1-25 EP 1743882 A1 KR 10-2007-0008455 A CN 1896052 A | | | |
| WO | 2015/060260 | A1 | 30 April 2015 | US 2016/0237198 A1 claims 1-33, paragraph [0045] EP 3061780 A1 KR 10-2016-0052619 A CN 105658694 A | | | |
| JP | 2019-099605 | A | 24 June 2019 | US 2020/0062976 A1 claims 1-9, paragraph [0122] WO 2018/110664 A1 EP 3556818 A1 | | | |
| JP | 2019-142029 | A | 29 August 2019 | (Family: none) | | | |
| JP | 2014-520086 | A | 21 August 2014 | WO 2012/160072 A1 claims 1-18 EP 2714650 A1 CN 103649044 A KR 10-2014-0037139 A | | | |
| JP | 2004-123666 | A | 22 April 2004 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006248998 A
- WO 2018190290 A
- WO 2011136345 A
- WO 2012067222 A
- JP 2021134198 A
- JP 2022043980 A

**Non-patent literature cited in the description**

- **C, NETSU.** *Sokutei,* 2012, vol. 39 (4), 143-150